# EUROPEAN PATENT SPECIFICATION

(11) **EP 2 384 709 B1**
(45) Date of publication and mention of the grant of the patent: **05.09.2012**
(21) Application number: 10162063.1
(22) Date of filing: 05.05.2010
(51) Int. Cl.: A61B 17/70, A61B 17/86, A61B 17/88

(54) **Receiving part for receiving a rod for coupling the rod to a bone anchoring element, bone anchoring device, method and tool for assembling the same**
Aufnahmeteil für die Aufnahme einer Stange zur Kopplung der Stange in einem Knochenverankerungselement, Knochenverankerungsvorrichtung, Verfahren und Werkzeug zu dessen Anordnung
Pièce de réception destinée à recevoir une tige pour coupler la tige à un élément d'ancrage d'os, dispositif d'ancrage d'os, procédé et outil d'assemblage

(43) Date of publication of application: 09.11.2011
(73) Proprietor: Biedermann Technologies GmbH & Co. KG, 78166 Donaueschingen (DE)
(72) Inventor: Biedermann, Lutz, 78048, VS-Villingen (DE); Dannecker, Berthold, 78112, St. Georgen (DE)
(74) Representative: Prüfer & Partner GbR European Patent Attorneys

(56) References cited:
- EP-A1- 2 022 423
- WO-A2-2007/038350
- US-A1- 2001 005 796
- US-A1- 2005 228 392
- US-A1- 2007 270 842
- US-A1- 2008 108 992

## Description

The invention relates to a receiving part for receiving a rod for coupling the rod to a bone anchoring element, a bone anchoring device with such a receiving part, a method and a tool for assembling the bone anchoring device. The receiving part includes a receiving part body and a locking ring. The locking ring can assume a first position, in which it is latched with respect to the receiving part body and in which a head of the bone anchoring element can be inserted, and a second position, in which it is latched with respect to the receiving part body and in which the bone anchoring element is held in an adjustable angular position but is not fully locked. The bone anchoring device can be realized, for example, in the form of polyaxial bone screw. The method of assembling includes the steps of providing the receiving part body and the locking ring in the first position, inserting the head and moving the locking ring into the second position. The tool is configured for the execution of the steps.

WO 2007/038350 A2 discloses an apparatus for connecting a bone anchor to a support rod, the apparatus including a connector body and a cap. The connector body has a socket for insertion, angulation and removal of a bone anchor. A sleeve is provided, which is configured to fit over the connector body in a temporary position, in which the sleeve permits insertion of the bone anchor, to move to a provisional locking position in which the sleeve permits angulation but prevents removal of the bone anchor, and to move to a locking position, in which the sleeve prevents both angulation and removal of the bone anchor.

< Insert from page la >

If the head of the anchoring element is freely pivotable with respect to the receiving part, the alignment of the receiving part and the insertion of the rod may be difficult in more complex clinical applications, for example, when a multitude of bone anchors have to be connected to the rod.

There is also a need to have the choice between different anchoring elements during surgery to select the most appropriate anchoring elements for the specific clinical application.

<EP 2 022 423 A1 shows a bone anchoring device comprising a bone anchoring element, a receiving part having a U-shaped recess; a locking ring wherein the head of the screw is locked by means of exerting pressure with the rod onto the locking ring resulting in compression of the receiving part and wherein the locking ring is mounted to the receiving part.

WO 2007/038350 A2 shows an apparatus for connecting a bone anchor to a support rod comprising a connector body having a channel to receive the support rod; and a cap to secure the support rod in the channel.

US 2005/0228392 A1 shows a pedicle screw comprising an anchoring member, a body and a fastening mechanism, wherein the fastening mechanism has an externally threaded set screw sized and configured to engage threads formed in the rod receiving channel so that tightening of the set screw secures the spinal rod within the rod receiving channel.

US 2008/0108992 A1 shows a multi-actual bone fixation device comprising a receiver defining an actual opening along a longitudinal axis; a bone fastener having a head; and a hollow member received around a second portion of the receiver wherein the hollow member compressing the second portion and retaining the head while allowing angulation of the fastener relative to the longitudinal axis.>

It is the object of the invention to provide an improved receiving part for receiving a rod for coupling the rod to a bone anchoring element, a bone anchoring device with such a receiving part which provides a safe handling during surgery and which allows to select an appropriate bone anchoring element and assemble it with the receiving part. It is further an object to provide a method for assembling the bone anchoring device which can be easily carried out and to provide a tool for assembly of the bone anchoring device.

The object is solved by a receiving part according to claim 1, a bone anchoring device according to claim 12, a method of assembling the bone anchoring device according to claim 13 and a tool according to claim 16. Further developments are given in the dependent claims.

The receiving part according to the invention allows the insertion of the head of the bone anchoring element into the receiving part body when the locking ring is in the first position which is an insertion position. In this position, it is latched with respect to the receiving part body. Therefore, the locking ring cannot be inadvertently moved to compress the head receiving portion of the receiving part body Therefore, the insertion of the head is facilitated.

In a second position, which is a pre-locking position, the locking ring is latched with respect to the receiving part body and the head receiving portion is compressed so that so that the bone anchoring element is held in an adjustable angular position but is not fully locked. This prevents inadvertent removal of the anchoring element and allows to hold the receiving part body with respect to the head of the bone anchoring element in an adjustable angular position. Therefore, a safe and convenient handling of the bone anchoring device during surgery can be assured.

In a third position, which is the locking position, the locking ring compresses the head receiving portion such that the bone anchoring element is fully locked and cannot pivot.

The receiving part body and the locking ring may be preassembled and may be delivered after manufacturing in a configuration in which the locking ring is latched in the first position to allow the introduction of the head of the screw element. A suitable bone anchoring element, for example a bone screw with a specific diameter and length can be selected and inserted into the receiving part. Thereafter, the locking ring can be moved into the second position with respect to the receiving part, in which the head is pre-locked. The latching of the locking ring in the receiving part body in the second position is audible, so that a person who assembles a bone anchoring device can be sure of a correct mounting of the screw element in the receiving part. In the pre-locked condition, the screw element is pivotable with respect to the receiving part only by applying an additional force to overcome the frictional force of the clamping of the head.

With the bone anchoring device a modular system can be provided, which allows to combine various anchoring elements with any suitable receiving part on demand depending on the actual clinical requirements. This reduces the costs of polyaxial screws, reduces the inventory and gives the surgeon a substantial choice of implants.

The method of assembling the bone anchoring device can be carried out by any specialist, for example, by the surgeon or any personnel assisting him before surgery.

The tool is easy to handle and enables a safe assembly.

The present invention relates to a device, a method and a tool for assembling a bone anchoring device, as claimed hereafter preferred embodiments of the invention are set forth in the dependent claims.

Further features and advantages of the invention will become apparent from the description of embodiments by means of the accompanying drawings.

In the drawings
- Fig. 1: shows a perspective exploded view of an embodiment of the bone anchoring device.
- Fig. 2: shows a perspective view of the bone anchoring device of Fig. 1 in an assembled state.
- Fig. 3: shows an enlarged perspective view of the locking ring.
- Fig. 4: shows a sectional view of the locking ring shown in Fig. 3 along line A-A in Fig. 3.
- Fig. 5: shows a sectional view of a bone anchoring device in an assembled state, the section being taken perpendicular to the rod axis, wherein the locking ring is in a first position in which it is latched with respect to the receiving part body.
- Fig. 6: shows a sectional view of the bone anchoring device in an assembled state, the section being taken in a plane perpendicular to the rod axis, wherein the locking ring is in a second position, in which it is latched with respect to the receiving part body.
- Fig. 7: shows a sectional view of the bone anchoring device in an assembled state with a rod inserted and fixed, the section being taken in a plane perpendicular to the rod axis, and wherein the locking ring is in a third position.
- Fig. 8: shows a sectional view of a portion of the bone anchoring device in a first step of assembling it, in which the bone anchoring element going to be inserted into the receiving part.
- Fig. 9: shows a sectional view of a portion of the bone anchoring device in second step of assembling it, in which the head has been introduced into the receiving part and is pre-locked.
- Fig. 10: shows a sectional view of a portion of the bone anchoring device, in which the head is locked.
- Fig. 11: shows an enlarged sectional view of a portion of the receiving part, wherein the locking ring is in the first position in which it is latched with respect to the receiving part body to allow the introduction of the head.
- Fig. 12: shows an enlarged sectional view of a portion of the bone anchoring device in the finally locked state of the head in which additional clamping is effected by means of the locking ring.
- Fig. 13: shows a perspective view of a tool for assembling the bone anchoring device.
- Fig. 14: shows an enlarged perspective view of a portion of the tool with the bone anchoring element inserted into a holder.
- Fig. 15: shows a perspective view of a portion of the tool with the receiving part to be inserted into a holder.
- Fig. 16: shows a perspective view of a portion of the tool with the receiving part introduced into a holder.
- Fig. 17a): shows a perspective view of the tool depicting a step of assembly, wherein the head of the bone anchoring element is going to be inserted into the receiving part.
- Fig.17b): shows an enlarged view of a portion of Fig. 17a).
- Fig. 18a): shows a perspective view of the tool with the head of the bone anchoring element inserted into the receiving part.
- Fig. 18b): shows an enlarged portion of Fig. 18a).
- Fig. 19: shows an enlarged perspective view of a portion of the tool after insertion of the head and before entering the pre-locking position of the locking ring.
- Fig. 20: shows a perspective view of the tool in a step of obtaining the pre-locking position of the locking ring.
- Fig. 21: shows a perspective view of an enlarged portion of the tool, wherein the locking ring has assumed the pre-locking position and clamps the head.

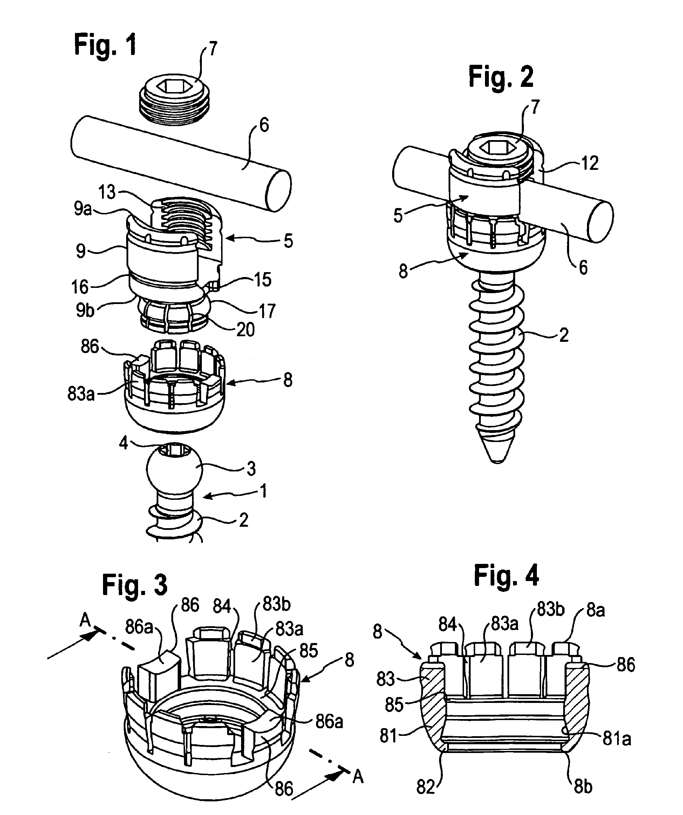

As shown in Figs. 1 to 7, the bone anchoring device comprises a bone anchoring element 1 in the form of a bone screw having a threaded shaft 2 and a head 3 with a curved surface portion. In this embodiment the head is spherical segment-shaped. The head 3 has a recess 4 for engagement with a tool. The bone anchoring device also comprises a receiving part body 5 for receiving a rod to connect it to the bone anchoring element 1. Further, a fixation element 7 in the form of an inner screw is provided for fixing the rod 6 in the receiving part body 5. The bone anchoring device includes a locking ring 8 for locking the head 3 in the receiving part body 5.

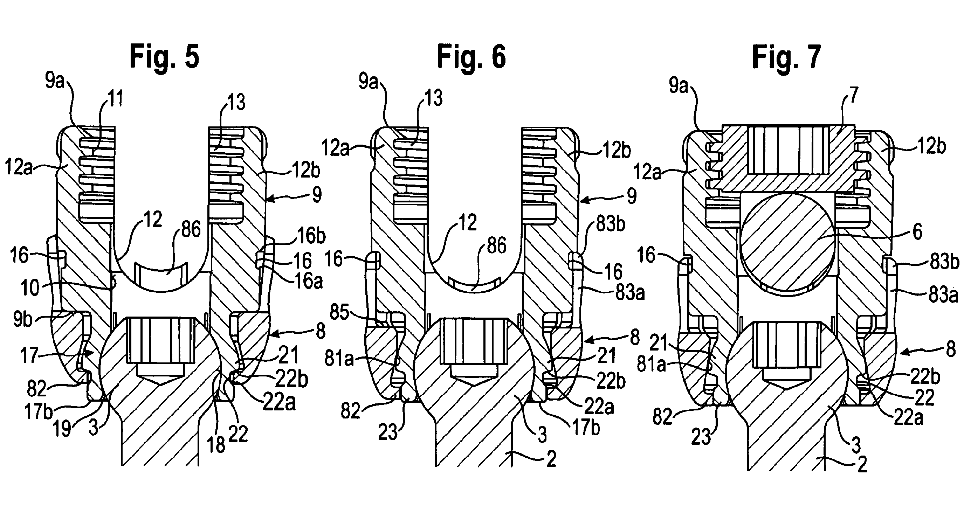

The receiving part body 5 comprises a rod receiving portion 9, which is substantially cylindrical and which has a first end 9a and an opposite second end 9b. A coaxial first bore 10 is provided at the second end 9b as shown in Figs. 5 to 7. The diameter of the first bore 10 is smaller than the diameter of the head 3 of the bone anchoring element. The rod receiving portion 9 further has a coaxial second bore 11 extending from the first end 9a to a distance from the second end 9b. The diameter of the second bore 11 is larger than that of the first bore 10. A substantially U-shaped recess 12 extends from the first end 9a in the direction of the second end 9b in the rod receiving portion, the diameter of the recess 12 being slightly larger than the diameter of the rod 6 in such a way that the rod 6 can be placed in the recess and can be guided therein. By means of the recess 12 two free legs 12a, 12b are formed on which an internal thread 13 is provided. The internal thread can be a metric thread, a flat thread, a negative angle thread, a saw-tooth thread, or any other thread form. Preferably, a thread form such as a flat thread or negative angle thread is used, which prevents splaying of the legs 12a, 12b when the inner screw 7 is screwed-in. The depth of the recess 12 is such that the rod 6 and the inner screw 7 can be inserted between the legs.

As can be seen in Fig. 1, cut-outs 15 are provided in the rod receiving portion on either end of the channel formed by the recess 12.

In the outer surface of the rod receiving portion 9 in the region of the legs 12a, 12b a groove 16 is provided, which extends in a circumferential direction and serves for engagement with a portion of the locking ring 8. The groove 16 is asymmetric in such a way that it allows a disengagement of the locking ring and the groove when the locking ring 8 is shifted in one direction. The asymmetric shape is realized by a downwardly inclined lower wall 16a and a substantially perpendicular upper wall 16b of the groove.

At the side of the second end 9b the receiving part body 5 comprises a head receiving portion 17 providing an accommodation space for the head 3 of the bone anchoring element 1. The head receiving portion 17 has a greatest outer diameter, which is smaller than the greatest outer diameter of the rod receiving portion 9. An internal hollow section 18 forms a seat for the head 3 of the bone anchoring element 1 and is open via the opening 19 to the free end 17b of the head receiving portion. The hollow section 18 is adapted in its shape to the shape of the head 3, in the embodiment shown, it is a spherical section to accommodate the spherical head 3. Furthermore, the hollow section 18 is configured to encompass the head 3 of the bone anchoring element from the side covering a region including the largest diameter of the head 3.

A plurality of slits 20 are provided in the head receiving portion 17, which are open to the free end 17b. The slits 20 render the head receiving portion 17 flexible so that it can be compressed to clamp and finally lock the head 3 in the hollow internal portion 18 by means of friction . The number and size of slits 20 is provided depending on the desired flexibility of the head receiving portion 17. The flexibility of the head receiving portion 17 is such that the head 3 of the anchoring element can be inserted by expanding the head receiving portion and that it can be clamped by compressing the head receiving portion.

The outer surface of the head receiving portion 17 has a first section 21, with an outer diameter which increases towards free end 17b, for example in an outwardly curved or conically widening manner. Adjacent to the first section 21, there is a circumferential groove 22, which is recessed with respect to the first section 21 and which serves for engagement with portion of the locking ring 8. The groove 22 is asymmetric to allow a disengagement of the locking ring and the groove when moving the locking ring in one direction. The asymmetric shape is realized by a lower downwardly inclined wall 22a and a substantially perpendicular upper wall 22 of the groove.

Adjacent the groove 22, there is a third portion 23 of the head receiving portion with a substantially cylindrical outer surface. The third portion 23 is configured to cooperate with a portion of the locking ring to enhance the clamping effect of the locking ring.

The locking ring 8 will now be described with reference to Figs. 1 to 7. The locking ring 8 is substantially cylindrical and has an upper end 8a and a lower end 8b. In the mounted state the upper end 8a is oriented in the direction of the first end 9a of the rod receiving portion and the lower end 8b is oriented towards the free end 17b of the head receiving portion. Near the lower end a first portion 81 with an inner surface 81a is provided, which cooperates with the first outer surface portion 21 of the head receiving portion to compress the head receiving portion. The outer surface of the first portion 81 may also be tapered to reduce the outer bottom diameter. The size of the first portion 81 is such that, for example, the tapered inner surface 81 a can engage the outer surface portion 21 of the head receiving portion 17 to exert a compression force onto the head receiving portion 17. The inner surface 81a of the first portion can also be curved with a curvature directing to the center of the locking ring.

At the lower end 8b, the locking ring comprises an inwardly projecting edge 82, the inner diameter of which is smaller than the inner diameter of the other portions of the locking ring. The inwardly projecting edge 82 is configured to engage the groove 22 of the head receiving portion.

The locking ring 8 further has a third portion 83 consisting of upwardly extending wall portions 83a, which are separated from each other by slits 84. The upwardly extending wall portions 83b are arranged at the outer circumference of an inner circumferential shoulder 85 of the locking ring and render the third portion 83 of the locking ring flexible. The number and size of the slits and the thickness of the wall portions 83a are configured such that a desired flexibility is obtained. At the free end the wall portions 83a comprise engagement sections 83b which are shaped so as to engage the groove 16 provided on the outer surface of the rod receiving portion. The inner diameter of the shoulder is only slightly larger than the outer diameter of the rod receiving portion 9 as can be seen in Fig. 5.

The locking ring 8 is sized in such a way with respect to the head receiving portion 17 that the head receiving portion can expand within the locking ring to allow the introduction of the head 3 when the locking ring is in the first position as shown in Fig. 5.

Two projections 86, which are located diametrically opposite to each other, are formed in the second portion 83 of the locking ring. The projections 86 have such a height that they project above the bottom of the substantially U-shaped recess 12 and extend into the cut-outs 15, when the locking ring 8 is in a position, in which the head 3 is not yet locked as shown in Figs. 5 and 6. The free end surface 86a of the projections 86 can be curved, particularly inwardly curved, with a curvature corresponding to that of the rod 6. The locking ring is arranged in such a manner around the head receiving portion 17 of the receiving part body 5, that the projections 86 are located at the positions of the recess 12. By means of this, the projections 86 prevent the locking ring from rotating when the rod is not inserted.

The flexibility of the head receiving portion 17 and the size of the head receiving portion at the open end 17b allows to mount the locking ring 8 by assembling it from the free end 17b onto the head receiving portion 17. Since the outer diameter of the head receiving portion is smaller than that of the rod receiving portion 9, the locking ring only minimally projects beyond the rod receiving portion in a radial direction.

The inner screw 7 has a thread corresponding to the internal thread 13 provided on the legs 12a, 12b. If a thread form, which prevents the legs from splaying is used, a single fixation element such as the inner screw 7 is sufficient. This reduces the size of the bone anchoring device in a radial direction. Other fixation elements such as, for example, an outer nut are also possible.

The receiving part body 5, the locking ring 8, the inner screw 7 and the bone anchoring element 1 are made of a bio-compatible material, for example of titanium or stainless steel or a bio-compatible alloy such as nitinol or a bio-compatible plastic material, such as PEEK. Parts can all be made of the same or of different materials.

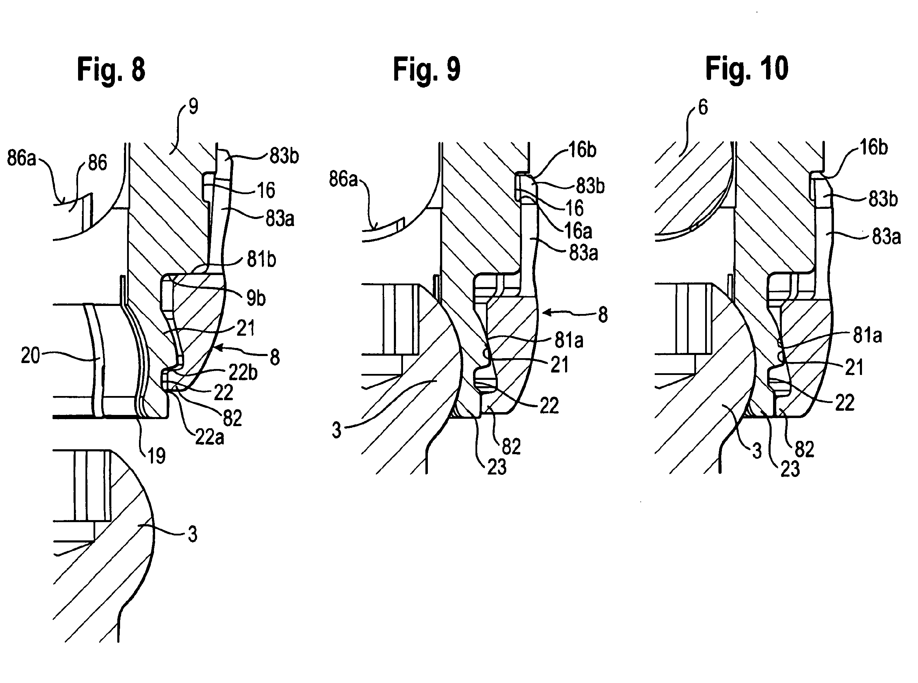

The function of the locking ring is now explained with referenced Figs. 5 to 12. As shown in Fig. 5, a first position of the locking 8, which is the insertion position and in which the locking ring 8 is latched with respect to the receiving part body 5, is defined in such a way that the inwardly projecting edge 82 engages groove 22 at the outer surface of the head receiving portion 17. As can be seen in the Figures, the inner diameter of the inwardly projecting edge 82 is larger than the outer diameter of the head receiving portion 17 at the position of the groove 22, so as to allow an expansion of the head receiving portion 17, when the head 3 is introduced. In the first position, the locking ring 8 is additionally held by a clamping force between the rod receiving portion 9 of the receiving part body 5 and the flexible wall portions 83a of the locking ring which are slightly bent outwards as can be seen in particular in Figs. 5 and 8.

When the locking ring is in the first position, the head receiving portion 17 is not compressed. In this position, the introduction of the screw head is possible as can be seen in Fig. 8. In the first position, the locking ring is prevented from moving upwards towards the first end 9a of the rod receiving portion, since it abuts with the shoulder 85 against the second end 9b of the head receiving portion and with the inwardly projecting edge 82 against the upper wall 16b of groove 16. As shown in particular in Fig. 8, the abutment of the locking ring against the second end 9b and against the upper wall of groove 16 holds the locking ring 8 in place against upward movement. The inclined lower wall 16a of the groove 16 prevents an inadvertent downward movement of the locking ring 8 but allows downward movement of the locking ring 8 upon exertion of an additional force. Since the inner diameter of the locking ring is larger than the outer diameter of the head receiving portion in a non-compressed state, an expansion of the head receiving portion 17 into the space within the locking ring is possible. In the first position, the head can freely pivot.

A second position, in which the locking ring is latched with respect to the receiving part body 5 and which is the pre-locking position is shown in Fig. 6, 9 and 11. In the second position, the locking ring 8 has been shifted towards the free end 17b of the head receiving portion until the engagement portions 83b of the flexible wall portions 83a resiliently snap into the groove 16 provided at the rod receiving portion 9. The free upper edge of the engagement portions 83b abut against the upper wall 16b of the groove 16, as shown in Figs. 6 and 9, thereby preventing upward movement of the locking ring out of the pre-locking position. On the other hand, the inclined lower wall surface 16a of the groove 16 prevents inadvertent downward movement of the locking ring towards the free end 17b but allows such downward movement upon exertion of an additional force.

In the second position, as can be seen in particular in Figs. 6 and 9, the inner inclined surface 81a of the locking ring presses against the first outer surface portion 21 of the head receiving portion so as to compress the head receiving portion 17 to clamp the head 3 within the hollow internal portion 18 without fully locking the head. In addition, the inwardly projecting edge 82 presses against the third portion 23 of the head receiving portion 17 resulting in an additional clamping force. Thereby, clamping of the head can be effected not only from above or the side of the head 3 but also from a region of the lower portion of head 3. Pre-locking means that under conditions arising during surgery, the angular position of the bone anchoring element 1 with respect to the receiving part body 5 is maintained and can be loosened only by exerting an additional force onto the receiving part body of the screw element. In the pre-locked position the bone anchoring element cannot be removed from the receiving part. Hence, an accidental or inadvertent removal of the head is not possible. However, angulation of the screw element is possible with an angle to be adjusted, for example manually.

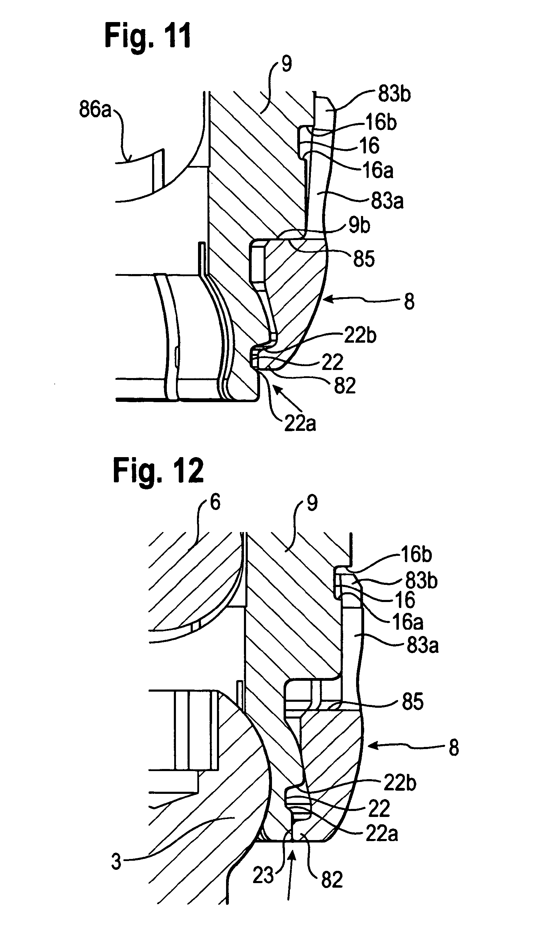

A third position, which is the locking position, is shown in Figs. 10 and 12. The third position is defined as the position, in which the screw head 3 is finally locked within the head receiving portion 17. The inner surface 81a of the locking ring engages the outer surface of the first portion 21 of the head receiving portion 17 in such a way that the head 3 is locked by compression of the head receiving portion. In addition, the inwardly projecting edge 82 further compresses the head receiving portion at the lower portion 23, thereby enhancing the locking force.

The dimensions of a receiving part body and the locking ring are configured such that a desired clamping force can be reached in the second position and in the third position, respectively.

The third position can be reached by shifting the locking ring relative to the receiving part body such that the engagement portions 83b and the inwardly projecting ring 82 slide along the lower inclined wall portions 16a and 22a of the grooves 16, 22, respectively.

The bone anchoring device is preassembled as follows. First, the locking ring 8 is mounted onto the receiving part body 5 from the free end 17b. This can be done, for example, by the manufacturer. Preferably, the locking ring 8 is in the first position in which it is latched by engagement of the inwardly projecting edge 82 with the groove 22. Thereafter, the head 3 of the anchoring element 1 is introduced from the free end 17b into the hollow internal portion 18 of the head receiving portion 17. Thereafter, the locking ring is moved relative to the receiving part body downwards, so that the inwardly projecting ring 82 slides out of the groove 22 and the engagement portions 83b of the flexible wall portions 83a snap into groove 16, whereby the second position, in which the head is pre-locked by frictional clamping is reached.

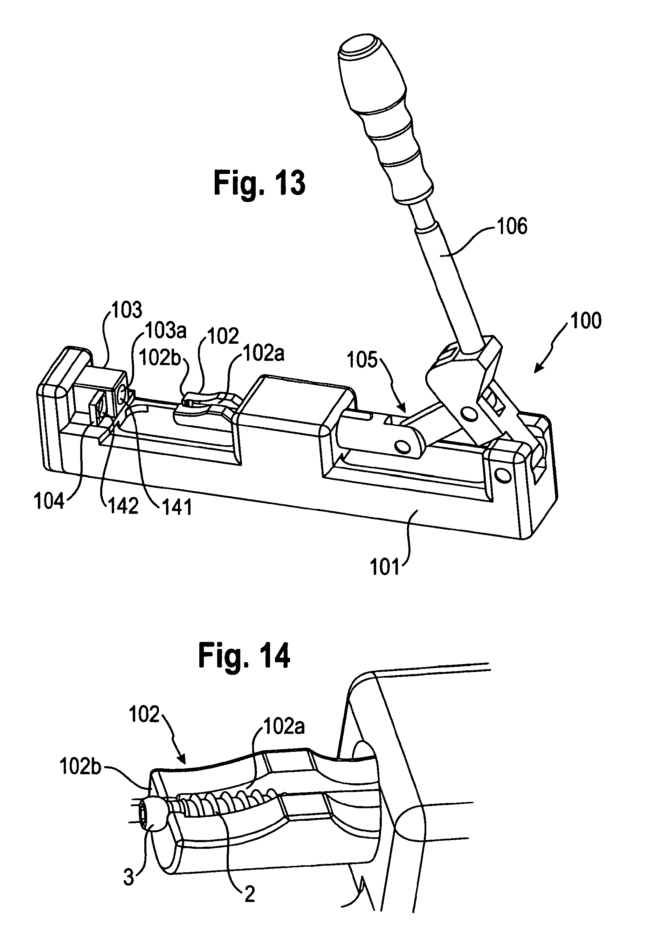

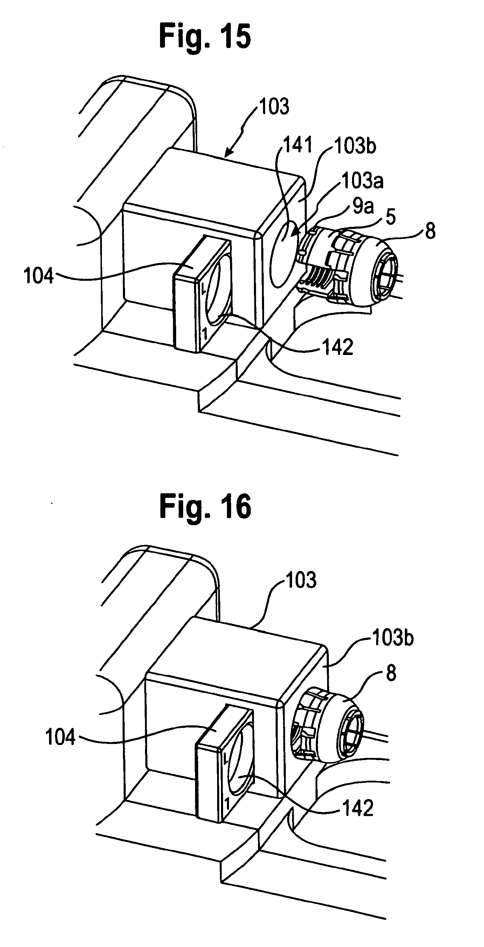

A tool for assembling the bone anchoring device and its operation will now be described with reference to Figs. 13 to 21. The tool 100 comprises a frame 101 with a first holder 102 for the screw element 1 and a second holder 103 for the receiving part. The holders 102, 103 are oriented such that the screw axis is horizontal with respect to the place on which the tool is standing. The holder 102 comprises a recess 102a for the shank 2 of the screw element, which serves for holding and guiding the shank 2. The diameter of the recess is smaller than the diameter of the head 3. By means of this, the free end surface 102b of the holder 102 serves as an abutment for the head 3 of the screw element 1. The holder 102 is supported on the frame 101.

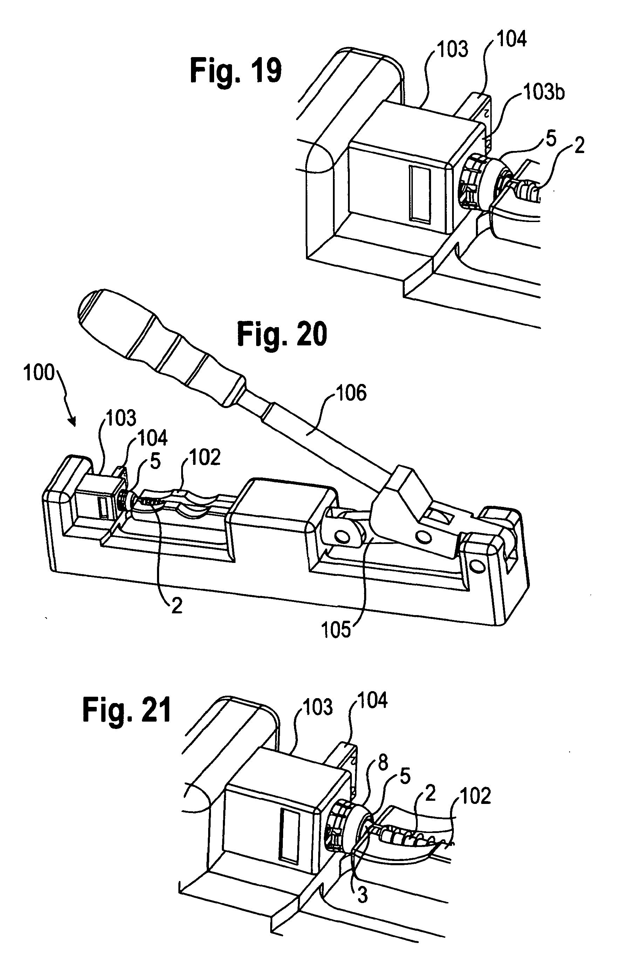

The holder 103 for the receiving part is also supported on the frame 101. It comprises a substantially circular recess 103a, for accommodating a portion of the receiving part. The orientation of the holder 103 with respect to the holder 102 is such that the central axis of the receiving part is coaxial to the screw axis when the receiving part and the screw element are both inserted into the holders. The circular recess 103a can have two different depths. This is realized by an insert 104 which is inserted in a corresponding slot provided in the holder 103 and which can be shifted in a direction transverse to the direction of the central axis of the recess 103a to limit the depth of recess 103a. The insert 104 has one circular recess 142. In position 1, as shown in Figs. 13 to 18, the insert limits the depth of the recess 103a to a first depth 141 and thus provides an abutment for the first end 9a of the receiving part. In position 2 which is shown in Figs. 19 to 21 the insert 104 is shifted such that its recess 142 forms the bottom of the recess 103a of the holder 103, the depth of which is greater than the depth 141 in the first position. The receiving part can be inserted deeper into the recess 103a until the outer surface 103b of the holder forms an abutment for the locking ring 8 as shown in Figs. 19 to 21. The shape of the recess 103a needs not to be circular but can be otherwise shaped, in particular, it can be adapted to the contour of the receiving part.

The holder 102 for the screw element is movable relative to the holder 103 for the receiving part body 5 in an axial direction. It can be actuated via a lever 105 and a handle 106. It has to be understood that the lever 105 is only an example and that the movement of the holder 102 for the screw element can be effected in many other ways.

The dimensions of the tool are configured such that by movement of the holder 102 for the screw element with respect to the holder 103, in which a receiving part is inserted, a sufficient force can be exerted to introduce the head of the screw element into the head receiving portion 17, when the recess 103a has the first depth 141 and the locking ring is in the first position. It is further configured such that a sufficient force can be exerted onto the locking ring, when holder 102 is moved again relative to the holder 103 to move the locking ring out of the first position into the second position, when the recess 103a has the second depth 142.

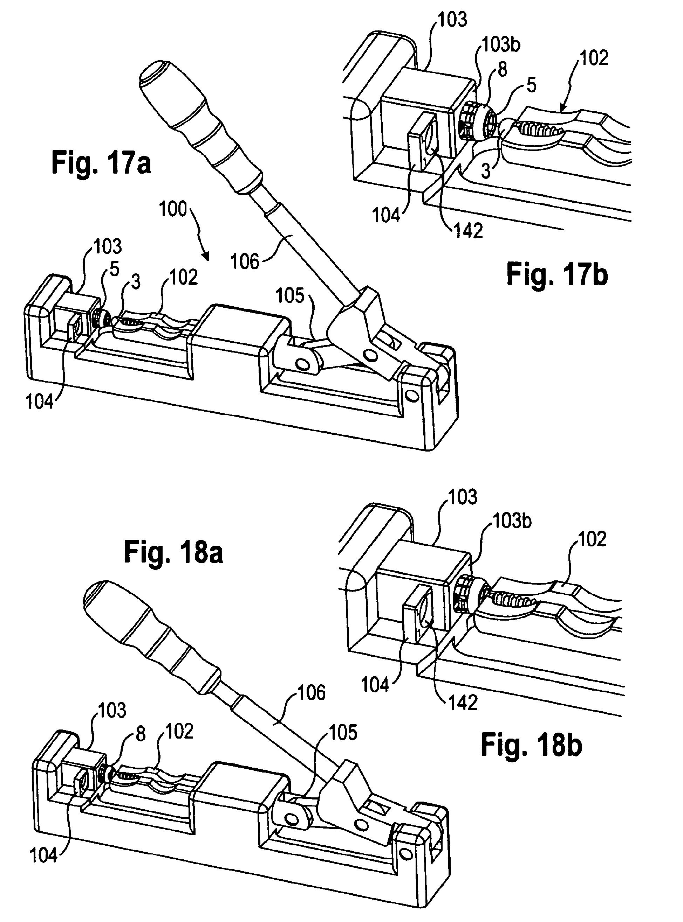

The operation of the tool is shown in Figs. 17 to 21. As shown in Figs. 17a) and 17b) first, the screw element 1 is inserted into the holder 102 and the receiving part body with mounted locking ring in the first position is mounted to the recess 103a of the holder 103 when the recess has the first depth 141.

In a next step, as shown in Figs. 18a) and 18b), the handle is actuated to actuate the lever 105 so that screw head 3 is pushed into the hollow internal portion 18 of the head receiving portion 17. The bottom of the recess 103a of the holder serves as an abutment for the receiving part, so that the head receiving portion 17 can expand to allow the introduction of the head 3. The handle is actuated until the head 3 of the screw element is latched in the hollow internal portion 18. The latching may produce an audible sign.

Thereafter, as shown in Figs. 19 to 21, the holder for the screw element is shifted backward and the insert 104 is moved to the second position, in which the circular recess 142 forms the bottom of recess 103 to provide an abutment for the locking ring 8.

As shown in Figs. 20 and 21, the handle 106 is actuated to push the holder 102 relative to the holder 103. By means of this, the head 3 with the receiving part body 5 is pushed against the bottom of the recess 103a, which has such a depth that the free front surface 103b of the holder 103 presses against the engagement portions 83b of the flexible wall portions 83a of the locking ring, thereby moving the locking ring out of the first position into the second position, in which it is latched in the groove 16 on the rod receiving portion 9. When the engagement portions 83b snap into the groove 16, the latching of the locking ring with respect to the receiving part body is audible, which indicates that the correct pre-locking position is reached.

Thereafter, the holder 102 is moved backward and the bone anchoring device is removed.

The bone anchoring device can be preassembled either by the manufacture or in the course of preparation of the surgery or at any other time. Advantageously, the surgeon selects prior to surgery the necessary receiving parts and bone anchoring elements according to the specific requirements of the clinical application. The design of the bone anchoring device allows to select the appropriate bone anchoring elements in terms of diameter, length and other features of the anchoring section. Hence, a modular system is provided, which includes receiving parts and several bone anchoring elements which then individually can be chosen and adapted.

In use during surgery, the preassembled bone anchoring device comprising the receiving part body, the bone anchoring element and the locking ring in the pre-locking position, is screwed into the bone. The recess 4 of the head can be accessed with a screw tool through the first bore 10. To correctly align the receiving part body with respect to the rod, to which it shall be connected, an additional force is exerted onto the receiving part either manually or by application of an instrument. Once the correct position of the rod with respect to other bone anchoring devices is achieved, the inner screw 7 is tightened. Since the rod 6 abuts onto the projections 86 of the locking ring, the locking ring 8 is shifted downward into the third position which is the locking position. When the locking ring 8 is moved towards the free end 17b of the head receiving portion, it compresses the head receiving portion, thereby locking the head. Final tightening of the inner screw locks the rod and the head simultaneously.

In the pre-locking condition, the head remains clamped when the inner screw is loosened. This allows further adjustments of the rod.

Further modifications of the embodiment shown are possible. For example, the head of the bone anchoring element can have any other shape, such as, for example, a cylindrical shape, whereby a monoaxial bone screw is provided allowing rotation of the screw element with respect to the receiving part body around a single axis. The head 3 can also be conically shaped or otherwise shaped and the internal hollow section 18 of the head receiving portion is adapted to this shape. In a further modification, the flexibility of the head receiving portion is based on properties of the material, for example a plastic material, and the slits may be fully or partly omitted.

The projections of the locking ring, which engage the rod, can have another shape, for example, the surface of the free end can be flat or can be otherwise shaped. The projections can be omitted.

The head receiving portion can have an inclined open end or can be otherwise asymmetric to allow a greater angulation of the head in one direction.

The outer surface of the head receiving portion and the inner surface of the locking ring can have other shapes which allow a compression of the locking ring by means of an increasing force when the locking ring is shifted downwards.

With regard to the tool, variations are also possible. For example, the tool can be configured such that the screw axis and the central axis of the receiving part extend perpendicular to the surface on which the tool is standing. The holder 103 for the receiving part body can be movable with respect to the holder 102 for the screw element. Instead of a manual actuation of the tool, it is possible to actuate the tool by means of a mechanically or electronically operated device.

## Claims

1. A receiving part for receiving a rod for coupling the rod to a bone anchoring element, the receiving part including
a receiving part body (5) with a top end (9a) and a bottom end (17b);
a rod receiving portion (9) with a channel (12) for receiving the rod (6), and
a head receiving portion (17) for accommodating a head (3) of the bone anchoring element, the head receiving portion having an open end (19, 17b) and being flexible so as to allow introduction and clamping of the head; and
a locking ring (8) embracing the head receiving portion (17), **characterized in that**
the locking ring (8) can assume a first position in which it is latched with respect to the receiving part body (5) and in which the head can be introduced into the head receiving portion (17) and
a second position in which it is latched with respect to the receiving part body (5) and compresses the head receiving portion such that the bone anchoring element is held in an adjustable angular position.

2. The receiving part of claim 1, wherein in the second position, the head is not fully locked.

3. The receiving part of claim 1 or 2, wherein the locking ring (8) can assume a third position with respect to the receiving part in which it compresses the head receiving portion (17) such that the head (3) is fully locked therein.

4. The receiving part of one of claims 1 to 3, wherein in the first position the locking ring (8) is prevented from moving in an axial direction towards the top end (9a) and can be moved towards to the bottom end (17b) by applying a force to loosen the latched state.

5. The receiving part of one of claims 1 to 4, wherein the locking ring (8) comprises a first projection (82) or a first recess which is configured to cooperate with a first recess (22) or a first projection at the receiving part body (5) to secure the first position.

6. The receiving part of claim 5, wherein the receiving part body comprises an asymmetric recess (22, 22a).

7. The receiving part of one of claims 1 to 6, wherein in the second position the locking ring is prevented from moving in an axial direction towards to the top end (9a) and can be moved towards the bottom end (17b) by applying a force to loosen the latched state.

8. The receiving part of claim 7, wherein the receiving part body comprises an asymmetric recess (16, 16a).

9. The receiving part of one of claims 1 to 8, wherein the locking ring has a first annular portion (81) facing the bottom end which is non-resilient and a second annular portion (83) facing the top end which is resilient.

10. The receiving part of one of claims 1 to 9, wherein the head receiving portion (17) has an exterior surface with a tapered or outwardly curved portion (21) and the locking ring (8) has an interior surface with a tapered or an inwardly curved portion (81a) which are configured to cooperate such that the head (3) is clamped when the locking ring is moved towards the bottom end.

11. The receiving part of claim 10, wherein the locking ring (8) has an inwardly projecting edge (82) at its lower end which cooperates with the head receiving portion (23) near the bottom end to provide additional clamping.

12. A bone anchoring device comprising a receiving part according to one of claims 1 to 11 and a bone anchoring element (1) having a shaft (2) with a threaded portion and a head with a spherical outer surface portion.

13. A method for assembling a bone anchoring device, the bone anchoring device including a receiving part for receiving a rod for coupling the rod (6) to a bone anchoring element, the receiving part comprising a receiving part body (5) and a locking ring (8), a bone anchoring element having a head which can be accommodated in the receiving part in a polyaxial manner and further having a shaft (2) for anchoring in the bone, the method including the steps:
providing the receiving part body with the locking ring in a first position in which it is latched with respect to the receiving part body and in which the head can be introduced into the receiving part body and can freely pivot therein;
inserting the head; and
moving the locking ring (8) and the receiving part body (5) relative to each other until the locking ring enters a second position in which it is latched with respect to the receiving part body and in which the bone anchoring element is held in an adjustable angular position.

14. The method of claim 13, wherein the bone anchoring device is a bone anchoring device of claim 12.

15. The method of claim 13 or 14, wherein the insertion of the head and/or the latching of the locking ring in the second position produces an audible sign.

16. Tool for assembling a bone anchoring device, the bone anchoring device comprising a bone anchoring element (1) with a head (3) and a shaft (2) to be anchored in the bone and a receiving part (5) according to one of claims 1 to 10, the tool comprising
a holder (102) for the bone anchoring element and
a holder (103) for the receiving part, which can be moved relative to each other to press the bone anchoring element (1) against the receiving part body (5) or the locking ring (8),
wherein the tool has an abutment exchange device (104), which allows the exchange of a first abutment (141) with a second abutment (142) to provide a first abutment (103a, 141) for the receiving part body (5) against movement of the screw element (1) and a second abutment (103a, 103b) for the locking ring (8) against movement of the receiving part body (5).

17. The tool of claim 16, wherein the holder (103) for the receiving part comprises a recess (103a) for inserting the receiving part body (5) and wherein the abutment exchange device (104) is configured to limit the depth of the recess to a first depth to provide the first abutment or to a second depth to provide the second abutment.

## Patentansprüche

1. Ein Aufnahmeteil zum Aufnehmen eines Stabs zum Koppeln des Stabs an ein Knochenverankerungselement, wobei das Aufnahmeteil umfasst:
einen Aufnahmeteilkörper (5) mit einem oberen Ende (9a) und einem unteren Ende (17b);
einen Stabaufnahmeteil (9) mit einem Kanal (12) zum Aufnehmen des Stabs (6) und
einen Kopfaufnahmeabschnitt (17) zum Aufnehmen eines Kopfs (3) des Knochenverankerungselements, wobei der Kopfaufnahmeabschnitt ein offenes Ende (19, 17b) hat und flexibel ist, um das Einführen und Klemmen des Kopfs zu erlauben; und
einen Verriegelungsring (8), der den Kopfaufnahmeabschnitt (17) umspannt, **dadurch gekennzeichnet, dass**
der Verriegelungsring (8) eine erste Position einnehmen kann, in der er bezüglich des Aufnahmeteilkörpers (5) eingeklinkt ist und in welcher der Kopf in den Kopfaufnahmeabschnitt (17) eingeführt werden kann und
eine zweite Position, in welcher er bezüglich des Aufnahmeteilkörpers (5) eingeklinkt ist und den Kopfaufnahmeabschnitt derart zusammendrückt, dass das Knochenverankerungselement in einer justierbaren Winkelposition gehalten ist.

2. Der Aufnahmeteil aus Anspruch 1, wobei der Kopf in der zweiten Position nicht vollständig verriegelt ist.

3. Der Aufnahmeteil aus Anspruch 1 oder 2, wobei der Verriegelungsring (8) bezüglich des Aufnahmeteils eine dritte Position einnehmen kann, in welcher er den Kopfaufnahmeabschnitt (17) derart zusammendrückt, dass der Kopf (3) vollständig darin verriegelt ist.

4. Der Aufnahmeteil aus einem der Ansprüche 1 bis 3, wobei der Verriegelungsring (8) in der ersten Position vom Bewegen in eine axiale Richtung zum oberen Ende (9a) hin abgehalten wird und durch Anlegen einer Kraft zum unteren Ende (17b) hin bewegt werden kann, um den eingeklinkten Zustand zu lösen.

5. Der Aufnahmeteil aus einem der Ansprüche 1 bis 4, wobei der Verriegelungsring (8) einen ersten Vorsprung (82) oder eine erste Ausnehmung aufweist, der/die konfiguriert ist, mit einer ersten Ausnehmung (22) oder einem ersten Vorsprung an dem Aufnahmeteilkörper (5) zusammenzuwirken, um die erste Position zu sichern.

6. Der Aufnahmeteil aus Anspruch 5, wobei der Aufnahmeteilkörper eine asymmetrische Ausnehmung (22, 22a) aufweist.

7. Der Aufnahmeteil aus einem der Ansprüche 1 bis 6, wobei der Verriegelungsring in der zweiten Position vom Bewegen in eine axiale Richtung zu dem oberen Ende (9a) hin abgehalten wird und durch Anlegen einer Kraft zu dem unteren Ende (17b) hin bewegt werden kann, um den eingeklinkten Zustand zu lösen.

8. Der Aufnahmeteil aus Anspruch 7, wobei der Aufnahmeteilkörper eine asymmetrische Ausnehmung (16, 16a) aufweist.

9. Der Aufnahmeteil aus einem der Ansprüche 1 bis 8, wobei der Verriegelungsring einen ersten ringförmigen Abschnitt (81) hat, der in Richtung des unteren Endes zeigt, der nicht federnd ist und einen zweiten ringförmigen Abschnitt (83), der in Richtung des oberen Endes zeigt, der federnd ist.

10. Der Aufnahmeteil aus einem der Ansprüche 1 bis 9, wobei der Kopfaufnahmeabschnitt (17) eine äußere Oberfläche mit einer kegeligen oder einem auswärts gebogenen Abschnitt (21) hat und der Verriegelungsring (8) eine innere Oberfläche mit einem kegeligen oder nach innen gebogenen Abschnitt (81a) hat, die konfiguriert sind derart zusammenzuwirken, dass der Kopf (3) geklemmt ist, wenn der Verriegelungsring zu dem unteren Ende hin bewegt wird.

11. Der Aufnahmeteil aus Anspruch 10, wobei der Verriegelungsring (8) eine nach innen vorstehende Kante (82) an seinem unteren Ende hat, die mit dem Kopfaufnahmeabschnitt (23) nahe des unteren Endes zusammenwirkt, um zusätzliche Klemmung bereitzustellen.

12. Eine Knochenverankerungsvorrichtung, die einen Aufnahmeteil gemäß einem der Ansprüche 1 bis 11 aufweist und ein Knochenverankerungselement (1) mit einem Schaft (2) mit einem Gewindeabschnitt und einem Kopf mit einem sphärischen äußeren Oberflächenabschnitt.

13. Ein Verfahren zum Montieren einer Knochenverankerungsvorrichtung, wobei die Knochenverankerungsvorrichtung umfasst:
einen Aufnahmeteil zum Aufnehmen eines Stabs zum Koppeln des Stabs (6) mit einem Knochenverankerungselement, wobei der Aufnahmeteil einen Aufnahmeteilkörper (5) und einen Verriegelungsring (8) aufweist, ein Knochenverankerungselement mit einem Kopf, der in einer polyaxialen Weise in dem Aufnahmeteil aufgenommen werden kann, und weiterhin mit einem Schaft (2) zum Verankern in den Knochen, wobei das Verfahren die Schritte umfasst:
Bereitstellen des Aufnahmeteilkörpers mit dem Verriegelungsring in einer ersten Position, in welcher er bezüglich des Aufnahmeteilkörpers eingeklinkt ist und in der der Kopf in den Aufnahmeteilkörper eingeführt werden kann und frei darin schwenken kann;
Einführen des Kopfs; und
Bewegen des Verriegelungsrings (8) und des Aufnahmeteilkörpers (5) relativ zueinander, bis der Verriegelungsring eine zweite Position einnimmt, in welcher er bezüglich des Aufnahmeteilkörpers eingeklinkt ist und in welcher das Knochenverankerungselement in einer justierbaren Winkelposition gehalten ist.

14. Das Verfahren aus Anspruch 13, wobei die Knochenverankerungsvorrichtung eine Knochenverankerungsvorrichtung aus Anspruch 12 ist.

15. Das Verfahren aus Anspruch 13 oder 14, wobei das Einführen des Kopfs und/oder das Einklinken des Verriegelungsrings in der zweiten Position ein hörbares Zeichen produziert.

16. Werkzeug zum Montieren einer Knochenverankerungsvorrichtung, wobei die Knochenverankerungsvorrichtung aufweist: ein Knochenverankerungselement (1) mit einem Kopf (3) und einem Schaft (2), um in den Knochen verankert zu werden und ein Aufnahmeteil (5) gemäß einem der Ansprüche 1 bis 10, wobei das Werkzeug aufweist:
einen Halter (102) für das Knochenverankerungselement
und
einen Halter (103) für das Aufnahmeteil, welche relativ zueinander bewegt werden können, um das Knochenverankerungselement (1) gegen den Aufnahmeteilkörper (5) oder den Verriegelungsring (8) zu drücken,
wobei das Werkzeug eine Widerlageraustauschvorrichtung (104) hat, welche den Austausch eines ersten Widerlagers (141) mit einem zweiten Widerlager (142) erlaubt, um ein erstes Widerlager (103a, 141) für den Aufnahmeteilkörper (5) gegen die Bewegung des Schraubenelements (1) bereitzustellen und ein zweites Widerlager (103a, 103b) für den Verriegelungsring (8) gegen die Bewegung des Aufnahmeteilkörpers (5)

17. Das Werkzeug aus Anspruch 16, wobei der Halter (103) für den Aufnahmeteil eine Ausnehmung (103a) zum Aufnehmen des Aufnahmeteilkörpers (5) aufweist und wobei die Widerlageraustauschvorrichtung (104) konfiguriert ist, die Tiefe der Ausnehmung auf eine erste Tiefe zu begrenzen, um das erste Widerlager bereitzustellen oder auf eine zweite Tiefe, um das zweite Widerlager bereitzustellen.

## Revendications

1. Partie de réception destinée à recevoir une tige pour le couplage de la tige à un élément d'ancrage osseux, la partie de réception comprenant
un corps (5) de la partie de réception avec une extrémité supérieure (9a) et une extrémité inférieure (17b) ;
une partie de réception de tige (9) avec un canal (12) pour recevoir la tige (6), et
une partie de réception de tête (17) pour recevoir une tête (3) de l'élément d'ancrage osseux, la partie de réception de tête ayant une extrémité ouverte (19, 17b) et étant flexible de manière à permettre l'introduction et le serrage de la tête ; et
un anneau de verrouillage (8) entourant la partie de réception de tête (17), **caractérisée en ce que**
l'anneau de verrouillage (8) peut prendre une première position dans laquelle il est verrouillé par rapport au corps (5) de la partie de réception et dans laquelle la tête peut être introduite dans le partie de réception de tête (17) ; et
une seconde position dans laquelle il est verrouillé par rapport au corps (5) de la partie de réception et comprime la partie de réception de tête de sorte que l'élément d'ancrage osseux soit maintenu dans une position angulaire réglable.

2. Partie de réception de la revendication 1, dans laquelle, dans la seconde position, la tête n'est pas entièrement verrouillée.

3. Partie de réception de la revendication 1 ou 2, dans laquelle l'anneau de verrouillage (8) peut prendre une troisième position par rapport à la partie de réception dans laquelle il comprime la partie de réception de tête (17) de sorte que la tête (3) y soit complètement verrouillée.

4. Partie de réception de l'une des revendications 1 à 3, dans laquelle dans la première position, l'anneau de verrouillage (8) est empêché de se déplacer dans une direction axiale vers l'extrémité supérieure (9a) et peut se déplacer vers l'extrémité inférieure (17b) en appliquant une force pour desserrer l'état verrouillé.

5. Partie de réception de l'une des revendications 1 à 4, dans laquelle l'anneau de verrouillage (8) comprend une première saillie (82) ou un premier évidement qui est configuré pour coopérer avec un premier évidement (22) ou une première saillie au niveau du corps (5) de la partie de réception pour fixer la première position.

6. Partie de réception de la revendication 5, dans laquelle le corps de la partie de réception comprend un évidement asymétrique (22, 22a).

7. Partie de réception de l'une des revendications 1 à 6, dans laquelle dans la seconde position, l'anneau de verrouillage est empêché de se déplacer dans une direction axiale vers l'extrémité supérieure (9a) et peut se déplacer vers l'extrémité inférieure (17b) en appliquant une force pour desserrer l'état verrouillé.

8. Partie de réception de la revendication 7, dans laquelle le corps de la partie de réception comprend un évidement asymétrique (16, 16a).

9. Partie de réception de l'une des revendications 1 à 8, dans laquelle l'anneau de verrouillage comporte une première partie annulaire (81) faisant face à l'extrémité inférieure qui est non-élastique et une seconde partie annulaire (83) faisant face à l'extrémité supérieure qui est élastique.

10. Partie de réception de l'une des revendications 1 à 9, dans laquelle la partie de réception de tête (17) présente une surface extérieure avec une partie conique ou incurvée vers l'extérieur (21) et l'anneau de verrouillage (8) présente une surface intérieure avec une partie conique ou incurvée vers l'intérieur (81a) qui sont configurées pour coopérer de sorte que la tête (3) soit serrée lorsque l'anneau de verrouillage se déplace vers l'extrémité inférieure.

11. Partie de réception de la revendication 10, dans laquelle l'anneau de verrouillage (8) présente un bord faisant saillie vers l'intérieur (82) à son extrémité inférieure qui coopère avec la partie de réception de tête (23) à proximité de l'extrémité inférieure pour fournir plus de serrage.

12. Dispositif d'ancrage osseux comprenant une partie de réception selon l'une des revendications 1 à 11 et un élément d'ancrage osseux (1) ayant un arbre (2) avec une partie filetée et une tête avec une partie de surface extérieure sphérique.

13. Procédé d'assemblage d'un dispositif d'ancrage osseux, le dispositif d'ancrage osseux comportant une partie de réception destinée à recevoir une tige de couplage de la tige (6) à un élément d'ancrage osseux, la partie de réception comprenant un corps (5) de la partie de réception et un anneau de verrouillage (8), un élément d'ancrage osseux ayant une tête qui peut être logée dans la partie de réception de manière polyaxiale et ayant en outre un arbre (2) pour l'ancrage dans l'os, le procédé comprenant les étapes consistant :
à doter le corps de la partie de réception de l'anneau de verrouillage dans une première position dans laquelle il est verrouillé par rapport au corps de la partie de réception et dans laquelle la tête peut être introduite dans le corps de la partie de réception et peut y pivoter librement ;
à insérer la tête ; et
à déplacer l'anneau de verrouillage (8) et le corps (5) de la partie de réception l'un par rapport à l'autre jusqu'à ce que l'anneau de verrouillage pénètre dans une seconde position dans laquelle il est verrouillé par rapport au corps de la partie de réception et où l'élément d'ancrage osseux est maintenu dans une position angulaire réglable.

14. Procédé de la revendication 13, dans lequel le dispositif d'ancrage osseux est un dispositif d'ancrage osseux de la revendication 12.

15. Procédé de la revendication 13 ou 14, dans lequel l'insertion de la tête et/ou le verrouillage de l'anneau de verrouillage dans la seconde position produit un signe sonore.

16. Outil d'assemblage d'un dispositif d'ancrage osseux, le dispositif d'ancrage osseux comprenant un élément d'ancrage osseux (1) avec une tête (3) et un arbre (2) à ancrer dans l'os et une partie de réception (5) selon l'une des revendications 1 à 10, l'outil comprenant
un support (102) pour l'élément d'ancrage osseux et
un support (103) pour la partie de réception, qui peuvent se déplacer l'un par rapport à l'autre pour presser l'élément d'ancrage osseux (1) contre le corps (5) de la partie de réception ou l'anneau de verrouillage (8),
où l'outil présente un dispositif d'échange de butées (104), qui permet l'échange d'une première butée (141) avec une seconde butée (142) pour fournir une première butée (103a, 141) pour le corps (5) de la partie de réception contre un mouvement de l'élément de vis (1) et une seconde butée (103a, 103b) pour l'anneau de verrouillage (8) contre un mouvement du corps (5) de la partie de réception.

17. Outil de la revendication 16, dans lequel le support (103) pour la partie de réception comprend un évidement (103a) pour l'insertion du corps (5) de la partie de réception et dans lequel le dispositif d'échange de butées (104) est configuré pour limiter la profondeur de l'évidement à une première profondeur afin de fournir la première butée ou à une seconde profondeur pour fournir la seconde butée.
